# FASCICULE DE BREVET EUROPEEN

(11) **EP 1 200 630 B1**
(45) Date de publication et mention de la délivrance du brevet: **17.11.2004**
(21) Numéro de dépôt: 00954455.2
(22) Date de dépôt: 10.07.2000
(51) Int. Cl.: C12Q 1/68

(54) **SYSTEM CAPTEUR BIOCHIMIQUE A SENSIBILITE ACCRUE PAR AMPLIFICATION MOLECULAIRE DU SIGNAL**
BIOSENSORSYSTEM MIT ERHÖHTER EMPFINDLICHKEIT MITTELS MOLEKULARER AMPLIFIKATION DES SIGNALS
BIOCHEMICAL SENSOR SYSTEM WITH INCREASED SENSITIVITY BY MOLECULAR AMPLIFICATION OF THE SIGNAL

(30) Priorité: 16.07.1999 FR 9909258
(43) Date de publication de la demande: 02.05.2002
(73) Titulaire: C.S.E.M. CENTRE SUISSE D'ELECTRONIQUE ET DE MICROTECHNIQUE SA, 2007 Neuchâtel (CH)
(72) Inventeur: SIGRIST, Hans, CH-3309 Kernenried (CH)
(74) Mandataire: Thérond, Gérard Raymond
(86) Numéro de dépôt international: PCT/EP2000/006513
(87) Numéro de publication internationale: WO 2001/006002

(56) Documents cités:
- EP-A- 0 669 395
- EP-A- 0 886 141
- WO-A-97/27317
- WO-A-98/58079

## Description

La présente invention a pour objet un système capteur biochimique dont la sensibilité est accrue par une amplification moléculaire d'un signal initialisé par l'interaction entre une entité biochimique présente dans une solution ou un fluide biologique et un réactif immobilisé sur le substrat du capteur et ayant une affinité spécifique pour ladite entité biochimique.

En matière de capteurs biologiques, on recherche de plus en plus des systèmes qui permettent de faire encore reculer les limites de détection et de dosage d'entités biochimiques dans des fluides biotiques, dans l'espoir d'obtenir une très grande sensibilité de détection. A cet effet, les perfectionnements technologiques ont porté non seulement sur l'environnement instrumental, par exemple sur les limites de détection d'un signal, mais aussi sur la conception même du capteur dès lors qu'on avait atteint les limites de sophistication au niveau instrumental. Mais là encore, les perfectionnements au niveau du capteur ont atteint un seuil au delà duquel il n'est plus possible de détecter des biomolécules à l'état de traces, seuil qui est de l'ordre du nanomolaire (nM) ou du picomolaire (pM).

Néanmoins d'autres perfectionnements ont permis de rendre le signal d'un capteur, jusqu'alors indétectable par les technologies antérieures, mesurable grâce à une amplification du signal sur lequel repose le principe de détection. Une telle amplification trouve son application préférée dans le domaine des capteurs biologiques étant donné que les conditions mises en oeuvre dans l'analyse biologique sont compatibles avec les systèmes de bioamplification.

Actuellement, deux modes de bioamplification sont employés dans des systèmes destinés à détecter, par exemple, des réactions immunologiques. Selon un premier mode d'amplification, dans les tests ELISA (Enzyme Linked Immunosorbent Assays), la molécule à détecter, par exemple un anticorps qui interagit avec une entité chimique tel qu'un antigène immobilisé, est chimiquement liée à une enzyme. L'enzyme sert à catalyser la transformation des molécules détectables. Dans les systèmes ELISA couramment utilisés, les enzymes qui catalysent la production d'entités chimiques sont presque toujours des hydrolases. Les produits de réaction solubles dans l'eau sont de préférence détectés dans l'ensemble du milieu réactionnel en mesurant l'absorption, la luminescence ou la bioluminescence.

Dans les biocapteurs, on obtient un deuxième mode d'amplification en augmentant le nombre ou la masse des espèces détectées. Ce principe d'amplification est réalisé par exemple en liant des marqueurs de masse à la molécule à détecter.

Si le principe de détection repose sur la fluorescence ou l'absorption, des molécules fluorescentes ou absorbantes sont chimiquement liées à l'entité chimique. A titre d'exemple d'amplification de ce type, on peut citer le brevet US 5,175,270 qui décrit un mécanisme d'amplification à partir d'une architecture dendrimère à la surface du capteur. La liaison de molécules modifiées sur chaque molécule cible, ou la liaison de réactifs secondaires marqués (par exemple des colloïdes, nanoparticules ou des anticorps secondaires marqués avec un fluorophore) produira une amplification linéaire du signal. Des billes de latex, des composés nanonocristallins semiconducteurs ou de l'or colloïdal sont des marqueurs de masse couramment utilisés dans les systèmes biocapteurs. Dans des systèmes d'amplification commerciaux, des anticorps secondaires fortement marqués par des molécules fluorescentes contribuent à augmenter le signal linéairement.

En général, les systèmes usuels amplifient les signaux du capteur par des réactions catalysées par une enzyme qui augmente le nombre d'entités chimiques secondaires de l'ensemble par catalyse (amplification catalytique pour une détection globale, cf. WO 9 727 317. Autrement, on augmente les signaux du capteur soit en ajoutant de la masse, pour une détection sensible à la masse, soit en augmentant le nombre de molécules marquées qui sont liées à l'unité.

A titre d'exemple d'amplification linéaire à la surface d'un capteur, on peut citer l'amplification d'un signal de fluorescence: dans ce système les anticorps secondaires sont conjugués pour permettre la détection de cibles en faible quantité.

Ces deux modes d'amplification qui viennent brièvement d'être rappelés ont permis d'augmenter sensiblement, soit en solution soit sur une surface, la sensibilité de détection : ils ne permettent cependant pas d'obtenir un signal suffisamment élevé pour les rendre suffisamment utilisables dans la pratique.

La présente invention a donc pour but d'augmenter encore le seuil de sensibilité d'un capteur biochimique par une méthode d'amplification n'ayant pas les inconvénients de l'art antérieur, en étant en particulier plus simple à mettre en oeuvre et moins coûteuse.

A cet effet, l'invention a pour objet un capteur biochimique à amplification moléculaire d'un signal pour la détection et le dosage d'une entité biologique en milieu biotique, cette entité biologique pouvant comprendre des oligonucléotides, des peptides ou des polysaccharides. Le système d'amplification est caractérisé en ce qu'on ajoute au milieu biotique des composés monomères et des unités catalytiques capables de catalyser à partir de l'extrémité d'un brin élémentaire de l'entité biologique un enchaînement polymère à partir desdits composés monomères en augmentant ainsi localement un paramètre physique mesurable à la surface du capteur.

Les unités catalytiques sont des enzymes choisies parmi toutes les classes de transférases, polymérases et synthétases qu'il est possible d'utiliser, soit individuellement, c'est-à-dire en ne choisissant qu'une seule espèce d'enzyme, soit en choisissant plusieurs enzymes qu'on utilise en combinaison ou qu'on ajoute de façon séquentielle au milieu biotique.

Parmi les unités catalytiques préférées, on peut citer une transférase spécifique à l'ADN ou à l'ARN simple qui allonge le brin oligonucléotide.

Les composés monomères, qui sont ajoutés au milieu biotique pour augmenter localement la masse par polymérisation, sont choisis de préférence parmi les acides nucléiques : NTP ou dNTP; où N = A (Adénosine), C (Cytidine), G (Guanosine) ou T (Tymidine) et d = déoxy.

Les peptidases, utilisées sous des conditions qui favorisent la réaction inverse, permettent la synthèse de matériaux protéiniques. Lorsqu'on veut obtenir une augmentation locale de masse des hydrates de carbone par une addition séquentielle d'enzymes, on choisit de préférence des monosaccharides transférases.

Comme indiqué précédemment, la détection et le dosage d'une entité chimique en milieu biotique repose fondamentalement selon l'invention sur une augmentation d'un paramètre mesurable, tel que la masse, à la surface même du capteur.

Selon un premier mode de détection, la surface du capteur a un agencement en réseau ou en gradient de réseau permettant de détecter, par exemple par des moyens optiques dans le champ d'évanescence une variation de l'indice de réfraction résultant de la variation de masse à la surface capteur, cette variation étant en corrélation avec le dosage de l'entité biochimique.

Selon un deuxième mode de détection, les composés monomères ajoutés au milieu biotique sont marqués avec un chromophore ou un fluorophore, de sorte que le polymère formé va augmenter localement la densité de marquage et permettre d'effectuer une mesure de fluorescence corrélable avec le dosage de l'entité biochimique.

Ces deux modes de détection sont donnés à titre d'exemples, mais le système capteur selon l'invention est adaptable à tout autre type de biocapteur sensible à une augmentation d'un paramètre physique, telle la masse à sa surface.

Il est donc nécessaire de maintenir à la surface du capteur le brin élémentaire de l'entité élémentaire permettant, par exemple, l'accroissement de masse par polymérisation à partir de l'une de ses extrémités. A cet effet, le substrat subit un traitement approprié, expliqué plus en détail par la suite, qui permet d'immobiliser directement ou indirectement l'unité de détection.

L'immobilisation directe de l'unité de détection s'effectue par une interaction covalente ou non covalente avec l'entité chimique à détecter, ladite interaction pouvant être unidirectionnelle en étant par exemple établie par une des extrémités de la séquence de nucléotides.

Cette immobilisation peut également être réalisée en utilisant un agent de réticulation photopolymérisable.

Lorsque l'immobilisation est effectuée de façon indirecte, on utilise une charpente moléculaire qui permet d'immobiliser un plus grand nombre d'unités de détection, ladite charpente moléculaire étant elle-même liée à la surface du biocapteur par une unité d'accrochage. De façon optimale, cette unité d'accrochage a une grande affinité pour interagir avec les molécules de détection. De telles interactions sont par exemple des interactions du type (premier anticorps)-(deuxième anticorps), comme c'est le cas dans les protocoles de test ELISA généralisés, ou des interactions de type ADN/ADN comme c'est le cas dans les dispositifs biocapteurs à base d'ADN.

De nombreuses structures peuvent être utilisées pour former la charpente moléculaire, parmi lesquelles on peut citer :
- de petites entités moléculaires qui permettent au moins une double fonctionnalisation, tel qu'un agent de réticulation hétéro-bifonctionnel par exemple N-(m-(triflurométhyl)diazirin-3yl)phényl)-4-maléimido-butyramide,
- un anticorps modifié avec un oligonucléotide ou un de ses fragments. La fraction hydrocarbonée et le domaine-clef des anticorps possèdent des groupes fonctionnels telles que des chaînes latérales carbohydrates et amino-acides qui respectivement facilitent l'addition d'oligonucléotides,
- des dendrimères ADN de tailles appropriées, qui présentent un grand intérêt en raison de leur aptitude à de multiples fonctionnalisations pour les oligonucléotides spécifiques. Des extensions à brin unique de dendrimères ADN permettent de fixer des anticorps fonctionnalisés à l'aide d'oligonucléotides,
- des colloïdes de métaux ou de composés nanocristallins semiconducteurs qui procurent les éléments essentiels pour une fonctionnalisation multiple.

L'unité d'accrochage qui établit une liaison sélective entre la charpente moléculaire et la molécule de détection est, dans un mode de réalisation préféré, formée par une partie d'une molécule identique à l'unité de détection, tel qu'un anticorps, ou par un fragment de celle-ci. Parmi les unités d'accrochage utilisables dans le cadre de la présente invention, on peut citer :
- toutes les classes d'immunoglobulines, la protéine A, la protéine G, la protéine fusionnée A-G,
- l'avidine, la neutravidine, la streptavidine et des oligonucléotides qui occupent un quart des sites individuels de liaison biotine,
- une polyhistidine marquée,
- un nitrolo-tetraacétate marqué,
- n'importe quel genre d'interaction moléculaire à liaison spécifique mais non covalente.

En fonction des caractéristiques de la charpente moléculaire, et notamment lorsqu'elle a une architecture dendrimère, l'unité de liaison polymère peut être un oligonucléotide avec une séquence de nucléotides en partie complémentaire de l'une des branches d'un dendrimère.

D'autres caractéristiques et avantages de l'invention apparaîtront dans la description suivante faite en référence aux dessins annexés dans lesquels :
- les figures 1 A, 1 B et 1 C représentent schématiquement les étapes conduisant à un premier mode de réalisation,
- la figure 2 est une représentation schématique d'un deuxième mode de réalisation, et
- les figures 3A et 3B montrent schématiquement un troisième mode de réalisation.

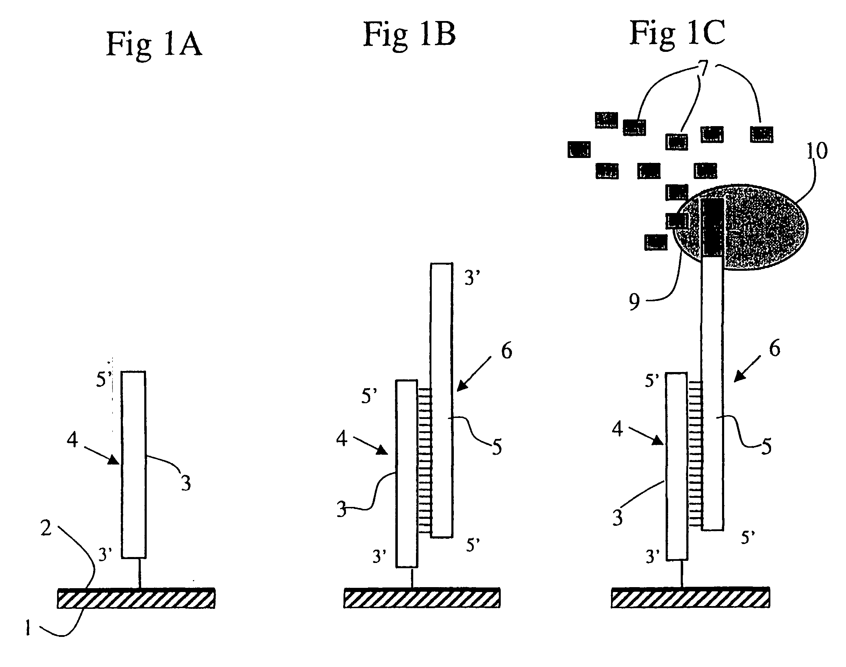

On va maintenant décrire un premier mode de réalisation d'un système capteur le plus simple selon l'invention, en référence aux figures 1A, 1B et 1 C où on a schématiquement représenté les étapes permettant d'amplifier un signal.

La première étape (figure 1A) montre un capteur 1 à la surface 2 duquel est immobilisé un oligonucléotide 3 par son extrémité 3'. Cet oligonucléotide 3 est désigné plus généralement par "molécule de détection" 4. Cette immobilisation peut être effectuée, soit par un traitement approprié de la surface du capteur pour permettre l'établissement d'une liaison covalente avec l'extrémité 3' de l'oligonucléotide 3, soit par un procédé thermochimique ou encore par une technique de photo-immobilisation au moyen d'un agent de réticulation photopolymérisable, comme cela sera expliqué plus en détail dans les exemples qui suivent. Cette molécule de détection comporte une séquence de nucléotides spécifique qui va permettre par hybridation (figure 1 B) d'immobiliser un brin élémentaire 5 de l'entité biochimique 6 à analyser, ce brin élémentaire ayant une séquence de nucléotides complémentaire de celle de la molécule de détection 4. Cette hybridation est effectuée en laissant libre l'extrémité 3' du brin élémentaire 5. Dans l'étape suivante (figure 1C), on a ajouté des nucléotides monomères 7, désignés par la suite par l'abréviation usuelle dNTP, et une enzyme 10, telle qu'une transférase à extrémité 3'. Cette enzyme 10 va catalyser spécifiquement la formation de liaisons covalentes entre l'extrémité 3' du brin élémentaire 5 et successivement les nucléotides ajoutés au milieu pour créer un enchaînement polymère 9 qui va augmenter la masse totale à la surface, ce qui va se traduire par une variation mesurable de l'indice de réfraction. Dans le cas où les nucléotides ajoutés au milieu sont marqués avec un marqueur fluorescent, cette augmentation de masse va se traduire par une accumulation de nucléotides marqués à la surface du capteur et une diminution globale de la fluorescence du milieu.

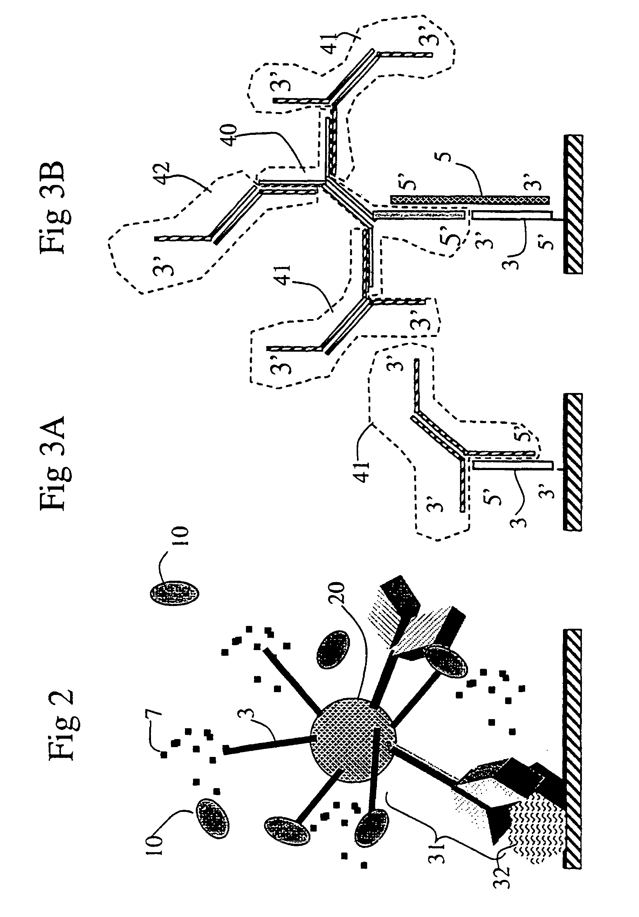

En se référant maintenant à la figure 2, on a représenté schématiquement un système capteur selon l'invention correspondant à un mode d'amplification plus complexe, en ce que les oligonucléotides 3 sont indirectement liées à la surface 2 du capteur 1 par l'intermédiaire d'une charpente moléculaire 20 immobilisée à la surface du capteur par une unité d'accrochage 30. Dans l'exemple représenté, la charpente moléculaire 20 est formée par un colloïde bifonctionnalisé pour retenir à la fois les oligonucléotides 3 attachés par l'extrémité 5' et un fragment d'anticorps 31 complémentaire d'un antigène 32 immobilisé à la surface, l'anticorps 31 et l'antigène 32 réalisant ensemble l'accrochage. Pour effectuer l'amplification du signal, on procède comme indiqué précédemment en ajoutant une enzyme 10 et des nucléotides 7.

La description ci-après donne en détail les différentes étapes qui permettent d'obtenir un système capteur biochimique selon l'invention et les modifications qu'il convient d'apporter à la surface d'un capteur optique du type de ceux qui reposent sur une détection de fluorescence ou sur une détection réfractométrique. Avec de petites modifications, les procédures décrites s'appliquent aussi à d'autres systèmes capteurs et à d'autres matériaux de transducteurs. Une modification de surface par silanisation convient aux surfaces qui donnent des groupes hydroxy de surface ou aux surfaces sur lesquels on peut produire des groupes hydroxy. D'une autre façon, on utilise aussi les technologies de photo-immobilisation pour fonctionnaliser une surface quand on souhaite avoir une fonctionnalisation de surface adressable et supprimer du milieu les liaisons non spécifiques de l'entité biologique. En principe, les matériaux employés pour le capteur sont des oxydes métalliques à la fois pour les mesures basées sur la réfractométrie et pour celles basées sur la fluorescence. Les modifications par charpente moléculaire amplificatrice ont été effectuées avec des colloïdes d'or et des dendrimères à plusieurs branches. Dans tous les exemples décrits ci-après, on a effectué l'amplification du signal avec la transférase à extrémité 3' (3'TT) pour catalyser l'addition de nucléotides à l'extrémité libre 3'.

### I. Fonctionnalisation de la surface du capteur et immobilisation de l'unité de détection

### Silanisation de la surface d'un oxyde métallique et liaison d'un oligonucléotide

En partant des protocoles de R. E. Kunz décrits dans les publications "Sensor and Actuators A (1997) 60,23" et "Sensors and Actuators B (1997) 38-39, 705", on a augmenté le nombre de radicaux hydroxy en traitant les capteurs optiques répliqués sur des polymères organiques avec un plasma d'oxygène dans un générateur de plasma. On a nettoyé les systèmes capteurs optiques sur verre par ultrasons dans l'acide nitrique 65% pendant trente minutes (30 mn), puis par rinçage à l'eau bidistillée. Les surfaces extemes d'oxyde métallique ont été silanisées en phase vapeur avec du 3-(glycidyloxy) propoyl-triméthoxysilane pendant deux jours à 180°C et 10 mbar. On a enfin immobilisé sur les surfaces époxy ainsi obtenues, des oligonucléotides commerciaux à chaîne terminale 3' ou 5'amino préalablement dissous dans un tampon de phosphate de sodium dilué à 1/100.

### Immobilisation d'oligonucléotides par l'intermédiaire de polymères photopolymérisables

Selon un protocole de photoimmobilisation de protéine décrit par H. Gao et al (Biotechnol. Appl. Biochem. (1994) 20, 251-263), la technique d'immobilisation adressable de biomolécules a été étendue à la liaison covalente d'oligonucléotides. Tout à la fois, le revêtement en couches et l'immobilisation en une seule étape se sont révélés applicables aux nucléotides. Au lieu d'utiliser de l'albumine de sérum bovin aryldiazirine modifié, on a employé à titre de nouveau réactif comme polymère photopolymérisable un dextrane aryldiazirine modifié. Un dextrane aryldiazirine modifié (T-Dextran) a été synthétisé par thiocarbamoylation de l'amino-dextrane avec la 3-(trifluorométhyl)-3-3(m-isothiocyanophényl) diazirine. Pour la photoimmobilisation des oligonucléotides, on a préparé une solution contenant 20 nanomoles de T-Dextran et 10 nanomoles d'oligonucléotides en solution dans un milieu tampon à pH 7,4 (1,5 mM de NaCl et 0,05 mM de phosphate de sodium).

On a utilisé ce mélange pour imprimer par jet d'encre une surface de 10 mm², ce qui correspond à une densité de 500 fmol/mm², soit encore 5 nl pour une surface de 3 x 3 mm. Une fois ce dépôt effectué, on séche les échantillons à température ambiante pendant 2 h à 20 mbar, puis on les expose à la lumière pour activer la réticulation du polymère. Cette immobilisation a été effectuée par une irradiation de trois minutes avec une source de lumière Orvel (11 mW/cm²) avec un filtre pour éliminer les radiations inférieures à 320 nm. Les surfaces modifiées sont lavées par plusieurs solutions tampons et finalement cinq fois à l'eau bidistillée. Par un marquage isotopique, on a pu déterminer que 40% des oligonucléotides étaient immobilisés sur le capteur, ce qui correspond approximativement à une densité de 200 fmol/mm².

### Immobilisation orientée d'oligonucléotides

Pour avoir une immobilisation orientée d'oligonucléotides, on a préparé des substrats ayant à leur surface du nitrure de silicium, des polymères organiques, du diamant ou encore du DLC (Diamond-like Carbon). Les substrats de base, à l'exception des polymères organiques, sont lavés aux ultrasons successivement 5 mn chaque fois dans de l'hexane et dans de l'éthanol et séchés pendant deux heures à température ambiante à 6 mbar. On dépose ensuite avec une seringue une goutte d'une solution éthanolique 0,25 mM de l'agent de réticulation N-(m-(triflurométhyl)diazirin-3yl)phényl)-4-maléimido-butyramide. Une goutte de 10 µl permet de couvrir une surface de 25 mm². Après séchage de deux heures à température ambiante à 30 mbar, on effectue la photoimmobilisation en irradiant les échantillons pendant 20 mn avec une source lumineuse Stratalinker à 350 nm procurant une irradiation de 0,9 mW/cm². On lave ensuite les surfaces modifiées trois fois avec de l'hexane et de l'éthanol; dans le cas d'un polymère organique utilisé comme substrat, on utilise le méthanol comme produit de lavage. Pour obtenir une immobilisation covalente, on dissout 10 nmol de 5'thio-oligonuléotide dans 50 µl d'une solution tampon dégazée à pH 7,7 (0,2 M HEPES + 1 mM EDTA). Cette solution est ensuite déposée par pipettage sur la surface maléimide modifiée et mise à incuber pendant seize heures à température ambiante. En dernier lieu, comme expliqué plus loin, on rince la surface oligonucléotide modifiée avec la solution tampon d'hybridation.

### II. Préparation de la charpente moléculaire

### Fonctionnalisation de colloïdes d'or avec des oligonucléotides et des fragments d'anticorps F(ab')

On effectue la sédimentation de colloïdes d'or de 20 nm de diamètre (10'000 tours/mn, pendant 30 mn) puis on élimine le surnageant et on ajoute une solution contenant 0,7 nmol de 5'thio-oligonucléotides et 0,3 nmol de fragments F(ab') fraîchement préparés dissous dans 50 µl d'une solution tampon dégazée à pH 7,7 (0,2 M HEPES + 1 mM EDTA). On garde la solution seize heures à température ambiante et on lave les colloïdes modifiés en effectuant trois cycles de sédimentation et de remise en suspension dans un tampon de phosphate de sodium.

### Charpente moléculaire de tri et multidentates d'ADN

Avec une structure ADN tridentée, on a un ADN à deux extrémités 3'OH et pour la première génération de dendrimère avec cinq extrémités 3'OH.

L'architecture dendrimère de cette charpente moléculaire a été préparée comme indiqué dans les brevets de la société Polyprobe (US 5,175,270; US 5,484,904; US 5,487,973). On a sélectionné des séquences d'oligonucléotides ADN en tenant compte d'une liaison efficace du brin complémentaire. On a assemblé (figure 3A) une structure dendrimère de base 41 en laissant libres les deux extrémités 3' pour l'amplification 3'TT, une extrémité 5' étant complémentaire de la molécule de détection immobilisée à la surface. Cet assemblage moléculaire permet de dupliquer le nombre de sites d'extension 3'TT. Les charpentes moléculaires de dendrimères de première génération ont été conçues de façon analogue. L'addition d'un dendrimère tétradenté (40) et d'un deuxième étage aux dendrimères tridentés (41) et bidentés (42) a porté le nombre de sites d'extension 3'TT à cinq (figure 3B).

### III. Hybridation de brins de ADN complémentaires et réaction de transférase à extrémité 3'

### Hybridation des oligonucléotides d'ADN avec les molécules de détection immobilisées à la surface

On a effectué la réaction d'hybridation avec les molécules de détection immobilisée à la surface, c'est-à-dire avec 15 à 60 nucléotides. Après immobilisation des molécules ADN de détection, on a lavé les surfaces trois fois avec une solution de 5xSSC contenant 0,1% en poids de dodecylsulfate de sodium pour éliminer les oligonucléotides adsorbés de façon non covalente. On a ensuite dissous des brins élémentaires d'ADN à chaîne unique dans 250 ml d'une solution d'hybridation comprenant en poids 1% de caséine, 0,1% de sel sodique de lauroylsarcosine et 0,02% de dodecysulfate de sodium dans une solution tampon de 5xSSC.

La solution ainsi obtenue a été déposée à la surface du capteur et on a laissé incuber deux heures à 45°C. Après l'étape d'hybridation, on a lavé les surfaces deux fois avec 2 x SSC contenant du dodécylsulfate de sodium à 0,1% en poids à température ambiante et trois fois avec une solution tampon 5 x SSG, contenant du dodecylsulfate de sodium à 0,1 % en poids, chauffée à 50°C. Ces opérations de lavage intensif incorporant des détergents sont nécessaires pour éliminer les brins élémentaires non hybridés.

### Réaction de la transférase à extrémité 3'(réaction 3'TT)

Dans l'étape suivante, on a ajouté aux brins élémentaires hybridés des nucléotides, ayant ou non un marqueur fluorescent, en même temps que l'enzyme 3'TT. Cette enzyme catalyse la liaison des nucléotides avec le brin élémentaire. En ajoutant des monomères aux brins élémentaires, l'enzyme augmente l'indice de réfraction pour la détection de masse ou la fluorescence à la surface du biocapteur. On a d'abord lavé la surface du capteur deux fois avec 200 µl du tampon cacodylate à pH 7 (0,5 M cacodylate, 5 mM CoCl₂, 1 mM dithiothreitol). On a fait démarrer la réaction en ajoutant deux unités de 3'TT à un milieu d'incubation composé de 20 µl de tampon cacodylate, 100 µl d'un déoxynucléotide phosphate (dNTP), 4 µl de 5 mM de dCTP et 100 µl d'eau. Le mélange a été brièvement mélangé par aspiration avec une pipette et déposé sur le substrat.

Les modifications d'indice de réfraction à la surface du capteur ont été suivies soit de façon optique avec un capteur optique intégré, soit par une détection de fluorescence. On a quantifié l'amplification du signal en effectuant la différence entre le signal le plus élevé obtenu d'une surface ayant le brin élémentaire immobilisé, et le signal provenant d'un capteur de référence dans lequel le brin élémentaire n'a subi aucune hybridation.

### IV. Amplification du signal dans un test génétique

### Test de détection de l'amplification du signal de fluorescence

Dans ce test, des molécules de détection d'ADN ont été immobilisées à la surface du capteur par photo-immobilisation à base de dextrane et hybridées avec les oligonucléotides qui sont, soit un composé témoin synthétique soit un oligonucléotide provenant de PCR. Dans le milieu réactionnel 3'TT, il y a des "dCTP" et des nucléotides fluorescents ChromaTide BODIPY FL-14-dCTP (Molecular Probes, Eugene Oregon, USA). On a initié la réaction d'amplification en ajoutant deux unités de 3'TT et on l'a arrêté après cinq minutes en lavant la surface du capteur avec la solution tampon de la réaction. On a suivi l'augmentation de fluorescence à la surface et comparé les résultats avec la fluorescence après hybridation d'une molécule de détection marquée à la fluoresceine.

Dans une deuxième série d'expériences, on a hybridé l'oligonucléotide à la fois à la branche de liaison de la première génération de dendrimères et à la molécule de détection liée à la surface. Avec cette configuration, la réaction 3'TT a lieu avec cinq extrémités 3' disponibles sur la charpente moléculaire.

On a enregistré l'incorporation des composés dCTP fluorescents. Les résultats expérimentaux montrent que le signal est augmenté d'un facteur 3,8 comparativement à celui d'un biocapteur agencé sans dendrimères.

### V. Amplification du signal 3'TT dans des tests immunologiques

On a examiné la liaison d'un anticorps à un antigène photoimmobilisé en utilisant la détection optique intégrée sans marqueur telle que décrite par H. Gao et al (Biosensors and Bioelectronics (1995) 10, 317-328). On a effectué le test avec un anticorps non modifié et, dans une deuxième série d'expériences, avec un anticorps fonctionnalisé avec un oligonucléotide. On a effectué la fonctionnalisation de l'anticorps comme indiqué par Ghosh et al (Bio-conjugate. Chem. (1990) 1,71-76). L'amplification du signal secondaire par la réaction 3'TT a été effectuée à la fois pour les deux systèmes de tests. Le milieu réactionnel comprenait des "dCTP" et deux unités de l'enzyme 3'TT. On a suivi en fonction du temps l'augmentation de masse en observant les modifications de l'indice de réfraction. La vitesse initiale de réaction et le niveau de saturation se sont révélés 64 fois plus grands avec l'échantillon comprenant l'anticorps modifié par les oligonucléotides qu'avec l'échantillon sans oligonucléotides.

On a atteint une augmentation d'un facteur 150 pour la sensibilité de détection d'une entité biologique avec une charpente moléculaire à colloïdes d'or bifonctionnalisée en ayant à sa surface à la fois des fragments d'anticorps spécifiques d'un antigène et des oligonucléotides. On a utilisé cet agencement moléculaire pour amplifier le signal d'un biocapteur immunologique. Comme décrit ci-dessus, on a modifié des particules d'or colloïdal de 10 nmoles avec des oligonucléotides et des fragments F(ab') dans une proportion de 7/3. On a ensuite appliqué à la surface du biocapteur ces colloïdes d'or bifonctionnalisés en même temps que deux unités 3'TT et on a enregistré l'augmentation de masse en fonction du temps. Le déterminant antigénique du fragment F(ab') immobilisé sur le colloïde s'oriente vers un épitope d'anticorps du test ELISA. L'amplification du signal en fonction de la masse a été obtenu d'abord au moyen d'une liaison sélective des colloïdes substitués. L'augmentation de la chaîne d'oligonucléotides provoquée par la réaction 3'TT conduit à une très forte augmentation du signal. Cela conduit finalement à l'amplification à saturation 150 fois supérieure à celle d'un échantillon sans colloïde.

## Revendications

1. Système capteur biochimique à amplification moléculaire d'un signal pour la détection et le dosage d'une entité biologique en milieu biotique, ladite entité biologique étant identifiable par au moins un brin élémentaire comprenant une séquence spécifique de nucléotides, ledit capteur ayant à sa surface une unité de détection immobilisée directement ou indirectement, ladite unité de détection possédant une séquence de nucléotides complémentaire de celle de l'entité biologique et ladite surface du capteur étant agencée pour délivrer à des moyens de détection et de mesure un signal représentatif d'une augmentation de masse par hybridation de l'entité biologique avec l'unité de détection, **caractérisé en ce que** le milieu biotique contient des composés monomères et des unités catalytiques capables de catalyser à partir de l'extrémité d'un brin élémentaire de l'entité biologique un enchaînement polymère à partir desdits composés monomères en augmentant ainsi localement la masse par une extension de la chaîne en produisant une amplification du signal mesurable à la surface du capteur.

2. Système capteur selon la revendication 1, **caractérisé en ce que** ledit signal mesurable est l'absorption d'une onde lumineuse ou l'émission d'un signal de fluorescence.

3. Système capteur selon la revendication 1, **caractérisé en ce que** les unités catalytiques sont des enzymes choisies parmi les transférases, les polymérases et les synthétases.

4. Système capteur selon la revendication 3, **caractérisé en ce que** les enzymes sont ajoutées au milieu biotique en choisissant une seule espèce, en combinant plusieurs espèces, ou en ajoutant séquentiellement plusieurs espèces.

5. Système capteur selon les revendications 3 ou 4, **caractérisé en ce que** l'enzyme est une transférase à brin ADN, telle qu'une transférase à extrémité 3', ou une polymérase à brin ARN.

6. Système capteur selon les revendications 3 ou 4, **caractérisé en ce que** l'entité biologique est un peptide ou une protéine et **en ce que** l'enzyme est une synthétase peptidée.

7. Système capteur selon la revendication 4, **caractérisé en ce que** l'entité biologique est un di-ou oligo-saccharide et **en ce que** les enzymes ajoutées de façon séquentielle comprennent une mono- ou oligo-saccharide transférase.

8. Système capteur selon l'une des revendications 1 à 5, **caractérisé en ce que** les composés monomères sont choisis parmi les nucléotides et les oligonucléotides

9. Système capteur selon l'une des revendications 1 à 8, **caractérisé en ce que** la surface du capteur a un agencement en réseau ou en gradient de réseau permettant une détection optique de la variation de l'indice de réfraction, liée à la variation de masse à la surface du capteur, cette variation d'indice de réfraction étant corrélable avec le dosage de l'entité biochimique.

10. Système capteur selon l'une des revendications 1 à 9, **caractérisé en ce que** les composés monomères sont marqués avec un chromophore ou un fluorophore permettant d'effectuer une mesure d'absorption ou de fluorescence corrélable avec le dosage de l'entité biochimique.

11. Système capteur selon l'une des revendications 1 à 10, **caractérisé en ce que** la séquence de nucléotides formant l'unité de détection est directement liée à la surface du capteur par liaison covalente.

12. Système capteur selon l'une des revendications 1 à 11, **caractérisé en ce que** l'unité de détection est liée de façon unidirectionnelle par son extrémité 3' ou 5'.

13. Système capteur selon l'une des revendications 1 à 12, **caractérisé en ce que** la séquence de nucléotide formant l'unité de détection est liée à la surface du capteur par photo-immobilisation.

14. Système capteur selon l'une des revendications 1 à 13, **caractérisé en ce que** la séquence de nucléotides formant l'unité de détection est indirectement liée à la surface du capteur par une charpente moléculaire bifonctionnelle, elle-même liée à ladite surface par une unité d'accrochage.

15. Système capteur selon l'une des revendications 1 à 14, **caractérisé en ce que** les composés permettant de former la charpente moléculaire sont choisis parmi, une entité moléculaire bifonctionnelle, tel qu'un agent de réticulation hétérobifonctionnel, un anticorps modifié par un nucléotide ou l'un de ses fragments, des dendrimères ADN de taille appropriée, et des colloïdes de métaux ou de composés nanocristallins semiconducteurs.

16. Système capteur selon la revendication 14, **caractérisé en ce que** les composés permettant de former l'unité d'accrochage sont choisis parmi les immunoglobulines, la protéine A, la protéine G, et la protéine fusionnée A-G.

17. Système capteur selon la revendication 14, **caractérisé en ce que** les composés permettant de former l'unité d'accrochage sont choisis parmi l'avidine, la neutravidine, la streptavidine et des oligonucléotides ADN ou ARN occupant un quart des sites de liaison biotine.

18. Système capteur selon la revendication 14, **caractérisé en ce que** les composés permettant de former l'unité d'accrochage sont choisis parmi une polyhistidine marquée et un nitroloacétate marqué.

19. Système capteur selon la revendication 14, **caractérisé en ce que** l'unité d'accrochage est formée par un oligonucléotide ayant une séquence de nucléotides partiellement complémentaire de l'une des branches d'un dendrimère lorsque la charpente moléculaire a une architecture dendrimère.

## Patentansprüche

1. Biochemisches Sensorsystem mit molekularer Verstärkung eines Signals für die Erfassung und die Dosierung einer biologischen Entität in einem biotischen Milieu, wobei die biologische Entität durch wenigstens einen elementaren Strang identifizierbar ist, der eine bestimmte Folge von Nukleotiden enthält, wobei der Sensor auf seiner Oberfläche eine direkt oder indirekt unbeweglich gemachte Erfassungseinheit aufweist, die eine Nukleotidfolge besitzt, die zu jener der biologischen Entität komplementär ist, und wobei die Oberfläche des Sensors so beschaffen ist, dass sie an Erfassungs- und Messmittel ein Signal liefert, das eine Zunahme der Masse durch Hybridisierung der biologischen Entität mit der Erfassungseinheit repräsentiert, **dadurch gekennzeichnet, dass** das biotische Milieu Monomer-Komponenten und katalytische Einheiten enthält, die beginnend bei einem Ende eines elementaren Strangs der biologischen Entität ausgehend von den Monomer-Komponenten eine Polymerkette katalysieren können, wodurch sie die Masse durch eine Verlängerung der Kette lokal erhöhen und dabei eine Verstärkung des an der Oberfläche des Sensors messbaren Signals erzeugen.

2. Sensorsystem nach Anspruch 1, **dadurch gekennzeichnet, dass** das messbare Signal die Absorption einer Lichtwelle oder die Emission eines Fluoreszenzsignals ist.

3. Sensorsystem nach Anspruch 1, **dadurch gekennzeichnet, dass** die katalytischen Einheiten Enzyme sind, die aus Transferasen, Polymerasen und Synthetasen gewählt sind.

4. Sensorsystem nach Anspruch 3, **dadurch gekennzeichnet, dass** die Enzyme dem biotischen Milieu hinzugefügt werden, indem eine einzige Sorte gewählt wird, mehrere Sorten kombiniert werden oder nacheinander mehrere Sorten hinzugefügt werden.

5. Sensorsystem nach den Ansprüchen 3 oder 4, **dadurch gekennzeichnet, dass** das Enzym eine Transferase mit ADN-Strang ist, etwa eine Transferase mit 3'-Ende oder eine Polymerase mit ARN-Strang.

6. Sensorsystem nach den Ansprüchen 3 oder 4, **dadurch gekennzeichnet, dass** die biologische Entität ein Peptid oder ein Protein ist und dass das Enzym eine Peptid-Synthetase ist.

7. Sensorsystem nach Anspruch 4, **dadurch gekennzeichnet, dass** die biologische Entität ein Di- oder Oligo-Saccharid ist und dass die nacheinander hinzugefügten Enzyme eine Mono- oder Oligo-Saccharid-Transferase umfassen.

8. Sensorsystem nach einem der Ansprüche 1 bis 5, **dadurch gekennzeichnet, dass** die Monomer-Komponenten unter den Nukleotiden und den Oligonukleotiden gewählt sind.

9. Sensorsystem nach einem der Ansprüche 1 bis 8, **dadurch gekennzeichnet, dass** die Oberfläche des Sensors eine Gitter- oder Gittergradienten-Anordnung aufweist, die eine optische Erfassung der Änderung des Brechungsindex, die mit der Massenänderung auf der Oberfläche des Sensors in Verbindung steht, ermöglicht, wobei diese Änderung des Brechungsindex mit der Dosierung der biochemischen Entität korrelierbar ist.

10. Sensorsystem nach einem der Ansprüche 1 bis 9, **dadurch gekennzeichnet, dass** die Monomer-Komponenten mit einem Chromophor oder mit einem Fluorophor markiert sind, was ermöglicht, eine Messung der Absorption oder der Fluoreszenz auszuführen, die mit der Dosierung der biochemischen Entität korrelierbar ist.

11. Sensorsystem nach einem der Ansprüche 1 bis 10, **dadurch gekennzeichnet, dass** die Folge von Nukleotiden, die eine Erfassungseinheit bildet, an die Oberfläche des Sensors durch Kovalenzbildung direkt gebunden ist.

12. Sensorsystem nach einem der Ansprüche 1 bis 11, **dadurch gekennzeichnet, dass** die Erfassungseinheit mit seinem 3'- oder 5'-Ende unidirektional gebunden ist.

13. Sensorsystem nach einem der Ansprüche 1 bis 12, **dadurch gekennzeichnet, dass** die Nukleotidfolge, die die Erfassungseinheit bildet, an die Oberfläche des Sensors durch Photo-Immobilisierung gebunden ist.

14. Sensorsystem nach einem der Ansprüche 1 bis 13, **dadurch gekennzeichnet, dass** die Nukleotidfolge, die die Erfassungseinheit bildet, an die Oberfläche des Sensors durch ein bifunktionales Molekulargerüst gebunden ist, das seinerseits an die Oberfläche durch eine Brückenbildungseinheit gebunden ist.

15. Sensorsystem nach einem der Ansprüche 1 bis 14, **dadurch gekennzeichnet, dass** die Komponenten, die die Bildung des Molekulargerüsts ermöglichen, gewählt sind aus einer bifunktionalen molekularen Entität wie etwa einem hetero-bifunktionalen Vernetzungsmittel, aus einem Antikörper, der durch ein Nukleotid oder eines seiner Fragmente modifiziert ist, aus ADN-Dendrimeren mit geeigneter Größe und aus Metall-Kolloiden oder aus nanokristallinen. Halbleiterkomponenten.

16. Sensorsystem nach Anspruch 14, **dadurch gekennzeichnet, dass** die Komponenten, die die Bildung der Brückenbildungseinheit ermöglichen, aus den Immunoglobulinen, dem A-Protein, dem G-Protein und dem verschmolzenen A-G-Protein gewählt sind.

17. Sensorsystem nach Anspruch 14, **dadurch gekennzeichnet, dass** die Komponenten, die die Bildung der Brückenbildungseinheit ermöglichen, gewählt sind aus Avidin, Neutravidin, Streptavidin und den ADN- oder ARN-Oligonukleotiden, die ein Viertel der Orte der Biotinbindung belegen, gewählt sind.

18. Sensorsystem nach Anspruch 14, **dadurch gekennzeichnet, dass** die Komponenten, die die Bildung der Brückenbildungseinheit ermöglichen, aus markiertem Polyhistidin und aus markiertem Nitroloacetat gewählt sind.

19. Sensorsystem nach Anspruch 14, **dadurch gekennzeichnet, dass** die Brückenbildungseinheit durch ein Oligonukleotid gebildet ist, das eine Nukleotidfolge besitzt, die zu einem der Zweige eines Dendrimers teilweise komplementär ist, wenn das Molekulargerüst eine Dendrimer-Architektur hat.

## Claims

1. Biochemical sensor system with molecular amplification of a signal for detecting and analysing a biological entity in a biotic medium, said biological entity being identifiable by at least an elementary strand including a specific nucleotide sequence, said sensor having at its surface a directly or indirectly immobilised detection unit, said detection unit having a complementary nucleotide sequence to that of the biological entity and said sensor surface being arranged to supply to detection and measuring means a signal representative of an increase of mass via hybridisation of the biological entity with the detection unit, **characterised in that** the biotic medium contains monomer compounds and catalytic units being capable of catalysing, from the end of an elementary strand of the biological entity, a polymeric concatenation of said monomer compounds thus locally increasing the mass by a chain extension inducing an amplification of the signal which can be measured at the sensor surface.

2. Sensor system according to claim 1, **characterised in that** said signal which can be measured is absorption of a light wave or emission of a fluorescence signal.

3. Sensor system according to claim 1, **characterised in that** the catalytic units are enzymes selected from among transferases, polymerases and synthetases.

4. Sensor system according to claim 3, **characterised in that** the enzymes are added to the biotic medium by selecting a single kind, by combining several classes or by adding several kinds sequentially.

5. Sensor system according to claim 3 or 4, **characterised in that** the enzyme is a DNA strand transferase, such as a transferase at end 3', or an RNA strand polymerase.

6. Sensor system according to claim 3 or 4, **characterised in that** the biological entity is a peptide or a protein and **in that** the enzyme is a peptided synthetase.

7. Sensor system according to claim 4, **characterised in that** the biological entity is a di- or oligo-saccharide and **in that** the sequentially added enzymes include a mono- or oligo-saccharide transferase.

8. Sensor system according to any of claims 1 to 5, **characterised in that** the monomer compounds are selected from among nucleotides and oligonucleotides.

9. Sensor system according to any of claims 1 to 8, **characterised in that** the sensor surface has a waveguide or waveguide gradient arrangement allowing optical detection of the refractive index variation, linked to the variation in mass at the sensor surface, this refractive index variation being able to be correlated with the analysis of the biochemical entity.

10. Sensor system according to any of claims 1 to 9, **characterised in that** the monomer compounds are labelled with a chromophor or a fluorophor allowing an absorption or fluorescence measurement to be made which can be correlated with the analysis of the biochemical entity.

11. Sensor system according to any of claims 1 to 10, **characterised in that** the sequence of nucleotides forming the detection unit is directly linked to the surface of the sensor by a covalent link.

12. Sensor system according to any of claims 1 to 11, **characterised in that** the detection unit is linked in a one-directional manner by its end 3' or 5'.

13. Sensor system according to any of claims 1 to 12, **characterised in that** the nucleotide sequence forming the detection unit is linked to the surface of the sensor by photo-immobilisation.

14. Sensor system according to any of claims 1 to 13, **characterised in that** the nucleotide sequence forming the detection unit is indirectly linked to the sensor surface by a bi-functional scaffold, which is itself linked to said surface by a docking unit.

15. Sensor system according to any of claims 1 to 14, **characterised in that** the compounds allowing the scaffold to be formed are selected from among, a bi-functional molecular entity, such as a hetero-bi-functional cross linking agent, an antibody modified by a nucleotide or one of its fragments, DNA dendrimers of suitable size, and metal or semiconductor nanocrystalline compound colloids.

16. Sensor system according to claim 14, **characterised in that** the compounds allowing the docking unit to be formed are selected from among immunogloblins, protein A, protein G and amalgamated protein A-G.

17. Sensor system according to claim 14, **characterised in that** the compounds allowing the docking unit to be formed are selected from among avidine, neutravidine, streptavidine and DNA or RNA oligonucleotides occupying a quarter of the biotin fink sites.

18. Sensor system according to claim 14, **characterised in that** the compounds allowing the docking unit to be formed are selected from among a labelled polyhistidine and a labelled nitroacetate.

19. Sensor system according to claim 14, **characterised in that** the docking unit is formed by an oligonucleotide having a partially complementary nucleotide sequence to one of the branches of a dendrimer when the molecular structure has a dendrimer architecture.
